# FASCICULE DE BREVET EUROPEEN

(11) **EP 1 928 408 B1**
(45) Date de publication et mention de la délivrance du brevet: **31.10.2012**
(21) Numéro de dépôt: 06808201.5
(22) Date de dépôt: 25.09.2006
(51) Int. Cl.: A61K 9/00, A61K 9/50, A61K 9/20, A61K 47/48, A61K 9/16

(54) **COMPRIMES ORODISPERSIBLES DE PRINCIPES ACTIFS AMERS**
IM MUND DISPERGIERBARE TABLETTEN MIT BITTEREN WIRKSTOFFEN
ORODISPERSIBLE TABLETS OF BITTER ACTIVE PRINCIPLES

(30) Priorité: 28.09.2005 FR 0509900
(43) Date de publication de la demande: 11.06.2008
(73) Titulaire: ETHYPHARM, 92213 Saint-Cloud Cédex (FR)
(72) Inventeur: CHAUDHARI, Mahendra, B., 400602 MAHARASHTRA (IN); Gendrot, Edouard, 2800 Garnay (FR)
(74) Mandataire: Cabinet Plasseraud
(86) Numéro de dépôt international: PCT/FR2006/002185
(87) Numéro de publication internationale: WO 2007/036632

(56) Documents cités:
- WO-A-00/40224
- WO-A-99/44580
- US-A- 3 594 470
- US-A- 5 219 563
- US-A- 5 271 946
- US-A1- 2005 036 977
- US-A1- 2005 181 050

## Description

La présente invention concerne des granulés enrobés contenant des principes actifs pharmacologiques dont les propriétés gustatives désagréables sont masquées à la fois par complexation par une résine échangeuse de cations et par enrobage. Elle concerne également des comprimés orodispersibles contenant de tels granulés enrobés.

Il est connu de complexer des substances pharmacologiquement actives présentant des qualités gustatives désagréables, telles qu'un goût amer, en les associant à des résines échangeuses de cations. Ainsi, les brevets US 6 193 962, US 5 811 436 et US 6 514 492 divulguent des formulations pharmaceutiques liquides contenant des principes actifs complexés par une résine de poly(acide méthacrylique) réticulée, en suspension dans une phase aqueuse. Le brevet US 5 219 563 divulgue des granules sèches non enrobés d'un principe actif amer (ranitine) complexé par du poly(acide méthacrylique) réticulé.

Dans le cadre de ses recherches visant à mettre au point des comprimés orodispersibles de substances actives dont il est nécessaire de masquer le goût désagréable, la Demanderesse a toutefois constaté que, pour un certain nombre de substances particulièrement amères et/ou laissant une sensation de brûlure dans la bouche, telles que la fluoxétine, le simple masquage du goût par complexation avec une résine échangeuse de cations était insuffisant.

Il est par ailleurs largement connu dans la technique de masquer le goût désagréable de certains principes actifs par enrobage avec des polymères gastrosblubles. Dans le cas des substances telles que la fluoxétine présentant une amertume très prononcée et laissant une sensation de brûlure dans la bouche, un tel masquage par enrobage est toutefois relativement inefficace et nécessite des quantités de polymère trop importantes. Ainsi, l'enrobage de la fluoxétine avec 50 % en poids de polymère suffit à peine à masquer l'amertume de la molécule et des quantités allant au-delà de cette valeur ne permettent pas d'obtenir une cinétique de dissolution en milieu acide conforme aux spécifications de la monographie USP des comprimés de fluoxétine qui exige un taux de dissolution supérieur à 85 % en poids en 15 minutes dans une solution d'HCl 0,1 N.

Il existe donc toujours un besoin pour des comprimés orodispersibles de substances pharmacologiquement actives ayant des qualités gustatives particulièrement désagréables, qui allient à la fois un bon masquage de goût et une libération rapide du principe actif en milieu acide.

La Demanderesse a constaté avec surprise qu'il était possible de préparer des formulations pharmaceutiques de principes actifs très amers et/ou provoquant une sensation de brûlure au niveau de la muqueuse buccale, qui permettent de masquer efficacement le goût du principe actif et se dissolvent rapidement en milieu acide, en complexant de manière connue le principe actif avec une résine échangeuse de cations faible comportant des groupes acide carboxylique et en effectuant la granulation du complexe ainsi obtenu en présence d'au moins 15 % en poids, rapporté au complexe résine/principe actif, d'au moins un adsorbant hydrophile, avant de soumettre les granulés ainsi obtenus à un enrobage par un polymère gastrosoluble.

La présente invention a par conséquent pour objet des granulés enrobés formés par un noyau comprenant un mélange
(A) d'au moins un principe actif pharmacologique aminé, de préférence sous forme de sel d'addition d'acide, ledit principe actif pharmacologique étant complexé par une résine échangeuse de cations faible comportant des groupes acide carboxylique (COO⁻), et
(B) d'au moins 15 % en poids, rapporté au poids total du complexe principe actif/résine échangeuse de cations faible, d'au moins un adsorbant hydrophile,
ledit noyau comprenant le mélange des composants (A) et (B) étant enrobé par un polymère gastrosoluble.

L'invention a en outre pour objet des comprimés orodispersibles comprenant de tels granulés enrobés et un ou plusieurs adjuvants choisis parmi les agents de désintégration, les diluants, les excipients pour compression directe, les agents d'écoulement, les lubrifiants, les édulcorants et les arômes.

Le principe actif qui peut être formulé sous forme de granulés et de comprimés orodispersibles selon la présente invention peut en principe être n'importe quelle molécule pharmacologiquement active hydrosoluble qui comporte une fonction amine permettant la formation de liaisons ioniques avec les groupes carboxyle de la résine échangeuse de cations. Il s'agit bien entendu de préférence d'un principe actif qui, lors de l'ingestion, présente des qualités gustatives désagréables nécessitant un masquage efficace. Comme expliqué en introduction, l'invention a un intérêt particulier pour des principes actifs très amers et/ou laissant une sensation de brûlure au niveau de la muqueuse buccale, tels que la fluoxétine, la rispéridone et la plupart des antiobiotiques, pour lesquels les méthodes de masquage de goût connues sont insuffisantes ou inadaptées.

Le principe actif est généralement utilisé sous forme de sel d'addition d'acide qui présente une meilleure solubilité dans l'eau que la forme non protonée, cette solubilité étant essentielle pour l'étape de complexation du principe actif par la résine échangeuse de cations qui se fait généralement en phase aqueuse. On peut utiliser en principe n'importe quel sel résultant de l'addition d'un acide pharmacologiquement acceptable connu. Un acide préféré est l'acide chlorhydrique.

La résine échangeuse de cations est une résine échangeuse de cations faible comportant des fonctions -COO⁻. Une telle résine présente l'avantage de retenir efficacement le principe actif complexé à un pH faiblement basique, neutre ou faiblement acide (pH de la salive : 7 ± 0,5), mais de le libérer rapidement dans un milieu fortement acide où la concentration élevée de protons déplace l'équilibre de dissociation de la résine échangeuse de cations vers la protonation des sites carboxyle.

Ces résines échangeuses de cations sont réticulées de manière à être insolubles dans tous les solvants, y compris l'eau, et à tous les pH. Elles ne sont pas absorbées par l'homme, ce qui fait d'elles des excipients non toxiques. Les résines échangeuse de cations utilisées dans la présente invention ont de préférence une capacité d'échange d'ions au moins égale à 5 mEq/g, de préférence au moins égale à 10 mEq/g et en particulier comprise entre 10 et 30 mEq/g.

On peut citer à titre d'exemples de résines échangeuses de cations faibles préférées, les copolymères d'acide méthacrylique et de divinylbenzène, commercialisées par exemple sous la dénomination AMBERLITE^{®} IRP88 de la société Röhm et Haas et sous la dénomination Indion 234 par Indion Resins.

Pour obtenir un masquage efficace du goût du principe actif, la résine échangeuse de cations est utilisée de préférence en une quantité pondérale au moins égale à celle du principe actif, et en particulier en une quantité pondérale supérieure à celle du principe actif. Des rapports en poids principe actif/résine compris entre 1 et 4, de préférence entre 1,5 et 2,5 ont donné des résultats satisfaisants. La préparation du complexe principe actif/résine échangeuse de cations sera décrite en détail ci-après et illustrée dans les exemples.

Comme indiqué ci-dessus, la granulation du complexe principe actif/résine échangeuse de cations doit se faire en présence d'une quantité relativement importante au moins égale à 15 % en poids, rapporté au poids total du complexe principe actif/résine échangeuse de cations faible, d'au moins un adsorbant hydrophile. Cette quantité importante d'adsorbant hydrophile permet, d'une part, d'adsorber l'eau en excès à l'issue de l'étape de complexation et l'utilisation d'un tel complexe principe actif/résine échangeuse de cations faible dans une formulation sèche telle qu'un comprimé, et favorise la dissolution rapide du principe actif complexé en milieu acide, malgré la double protection, par complexation et enrobage, qui a normalement tendance à retarder la libération du principe actif.

De préférence, dans le cas de la fluoxétine, la présence d'adsorbant hydrophile en quantité au moins égale à 15% en poids, rapporté au poids total du complexe principe actif/résine échangeuse de cations faible, permet d'obtenir une cinétique de dissolution en milieu acide conforme aux spécifications de la monographie USP des comprimés de fluoxétine qui exige un taux de dissolution supérieur à 85 % en poids en 15 minutes dans une solution d'HCl 0,1 N.

On utilise n'importe quel adsorbant hydrophile ou n'importe quelle association d'adsorbants hydrophiles choisi(e) dans le groupe consistant en la cellulose microcristalline, les amidons, le lactose, le lactose monohydrate, la silice colloïdale telle que l'Aérosil, et la silice précipitée telle que la Syloid 244 FP. Dans un mode de réalisation préféré des granulés selon l'invention, ceux-ci contiennent de 15 à 50 % en poids, rapporté au poids total du complexe principe actif/résine échangeuse de cations faible, et en particulier de 20 à 40 % en poids, d'au moins un adsorbant hydrophile.

Dans un mode de réalisation préféré, on utilise en tant qu'agents hydrophiles l'association d'une cellulose microcristalline et d'une silice colloïdale.

Dans un mode de réalisation particulièrement préféré de l'invention, le complexe principe actif/résine échangeuse de cations est granulé en présence d'au moins 15 % en poids de cellulose microcristalline et d'au moins 5 % en poids de silice colloïdale.

Les granulés obtenus contenant à la fois le complexe principe actif/résine échangeuse de cations faible et au moins 15 % en poids, rapporté au poids total du complexe principe actif/résine échangeuse de cations, d'au moins un adsorbant hydrophile, sont enrobés par un ou plusieurs polymères gastrosolubles en une quantité suffisante pour masquer efficacement le goût d'éventuels résidus de principe actif non complexés par la résine échangeuse de cations.

On entend par polymère gastrosoluble selon la présente invention tout polymère insoluble dans un milieu aqueux basique ou neutre et qui, lorsqu'il est mis en contact avec le milieu acide de l'estomac présentant généralement un pH compris entre 1 et 3, se dissout totalement dans celui-ci.

Les polymères gastrosolubles sont connus et peuvent être choisis par exemple parmi les dérivés cellulosiques et les polymères (méth)acryliques. On peut citer à titre d'exemples de dérivés cellulosiques appropriés l'éthylcellulose, l'hydroxypropylméthylcellulose et l'hydroxypropylcellulose. Les polymères (méth)acryliques utilisables en tant que polymères gastrosolubles pour l'enrobage des granulés sont par exemple l'EUDRAGIT E 100 (copolymères de méthacrylates d'alkyle et de méthacrylate d'aminoalkyle).

La quantité de polymère gastrosoluble nécessaire pour obtenir un masquage satisfaisant du goût résiduel est généralement compris entre 5 et 50 %, de préférence entre 10 et 40 % et en particulier entre 15 et 30 % en poids, rapporté au poids total des granulés avant enrobage. L'enrobage peut se faire selon des techniques connues, par exemple par pulvérisation d'une solution aqueuse et/ou alcoolique du polymère sur des granulés en suspension dans un lit fluidisé.

L'invention a enfin pour objet un procédé de fabrication de granulés enrobés comprenant,
(a) la complexation, en milieu aqueux, d'au moins un principe actif pharmacologique aminé par une résine échangeuse de cations faible comportant des groupes carboxyle (-COO⁻),
(b) la granulation du complexe principe actif/résine obtenu contenant encore au moins une partie de l'eau utilisée pour la complexation, en présence d'au moins 15 % en poids, de préférence de 15 à 50 % en poids, rapporté au poids total du complexe principe actif/résine, d'au moins un adsorbant hydrophile, et
(c) l'enrobage des granulés obtenus à l'étape (b) par au moins un polymère gastrosoluble.

La complexation du ou des principes actifs par la résine échangeuse de cations se fait par simple mise en contact de ces deux composants dans un milieu aqueux sous agitation. On procède généralement et de préférence en dispersant d'abord le principe actif dans le milieu aqueux afin de le dissoudre au moins partiellement. La résine échangeuse de cations est ensuite ajoutée en une seule fois ou en plusieurs portions et l'ensemble est agité pendant un temps suffisant pour obtenir la complexation de la quasi-totalité du principe actif. Le processus de complexation est relativement lent car le principe actif dissous doit pénétrer dans les particules de résine insolubles, gonflées par le solvant aqueux. Le temps d'agitation du mélange nécessaire pour obtenir une complexation de la quasi-totalité du principe actif est généralement au moins égal à 1 heure, de préférence compris entre 2 et 4 heures. Dans le cas de la complexation de la fluoxétine par une résine de poly(acide méthacrylique) réticulée, la Demanderesse a constaté qu'au bout de 2 heures, 98 % du principe actif avaient été complexés par la résine. Des tests en bouche ont toutefois montré qu'il était préférable de prolonger la durée de complexation jusqu'à 3 heures. Dans un mode de réalisation alternatif du procédé de l'invention, la complexation peut se faire en deux étape : une première étape de complexation avec une fraction de la résine, puis filtration de la suspension et mise en contact de la fraction restante de résine avec le filtrat contenant une faible concentration de principe actif non encore complexé.

A la fin de l'étape de complexation, le complexe principe actif/résine n'est pas séché mais soumis à l'étape de granulation suivante avec au moins une partie de l'eau utilisée pour l'étape de complexation. Une partie de l'eau de complexation, comprise généralement entre 30 et 70 %, est éliminée par une technique appropriée telle que la filtration, la décantation, la sédimentation par centrifugation ou l'essorage, ou par une combinaison de ces techniques. La Demanderesse a notamment mis au point une technique d'essorage du complexe par centrifugation dans un sac de filtration (voir exemple 1) qui permet d'éliminer environ 50 % en poids de l'eau et qui est considérablement plus rapide que la méthode de décantation suivie de l'aspiration du surnageant utilisée jusqu'ici.

Le ou les adsorbants hydrophiles sont ensuite mélangés au complexe encore humide et l'ensemble est soumis à une granulation selon un procédé connu.

L'enrobage des granulés ainsi obtenus peut se faire également selon n'importe quelle technique appropriée connue, par exemple par pulvérisation d'une solution du polymère gastrosoluble, éventuellement additionné d'un ou plusieurs adjuvants, sur les granulés mis en suspension dans un lit fluidisé.

La préparation d'un comprimé orodispersible selon l'invention à partir des granulés obtenus de la manière décrite ci-dessus se fait de manière connue (voir par exemple les demandes de brevet français FR2679451, FR2766086, FR2785538, FR2790387, FR2831820 de la Demanderesse) par mélange des granulés enrobés décrits ci-dessus avec un ou plusieurs adjuvants connus, choisis par exemple parmi les agents de désintégration, les diluants, de préférence les diluants solubles tels que des sucres ou polyols, les excipients pour compression directe, les agents d'écoulement, les lubrifiants, les édulcorants et les arômes, et compression à sec dans une presse à comprimés.

L'invention est illustrée ci-après à l'aide des exemples de réalisation suivants.

### Exemple 1

### Préparation de comprimés orodispersibles de fluoxétine

### Complexation (étape (a) du procédé)

On pèse, puis on soumet à un tamisage (tamis 630 µm) 100 g de chlorhydrate de fluoxétine, 200 g de résine AMBERLITE IRP88 (poly(acide méthacrylique) réticulé par divinylbenzène). On met 800 g d'eau purifiée sous agitation puis on y introduit lentement la fluoxétine sur une période d'environ 5 minutes et l'on poursuit l'agitation du mélange pendant environ 5 minutes, avant d'ajouter progressivement, sur une durée d'environ 10 minutes, l'AMBERLITE IRP88. On observe alors un épaississement de la suspension qui se refluidifie toutefois assez rapidement. On maintient le mélange ainsi obtenu pendant 2 heures sous agitation en prenant soin de limiter la vitesse d'agitation à la valeur minimale tolérée par l'agitation afin d'éviter la formation de bulles.

### Essorage du complexe formé

On verse le mélange obtenu dans l'étape (a) ci-dessus dans un sac de filtration en polypropylène (porosité 1 - 5 µm) placé dans un panier d'une essoreuse (essoreuse Rousselet RC 20) mis en rotation à une vitesse de 400 tours/min. On récupère le filtrat, qui est une suspension trouble et blanchâtre de particules très fines, et on le verse de nouveau dans le sac de filtration contenant le retentat. Ce recyclage du filtrat est effectué deux fois, avec augmentation de la vitesse de l'essoreuse entre 500 et 1000 tours/min entre chaque recyclage. Après le dernier recyclage du filtrat, on fait tourner l'essoreuse pendant quelques minutes. L'essorage est arrêté quand le filtrat est recueilli à une cadence d'une goutte par seconde. On obtient ainsi un retentat dont environ 53 % de l'eau initialement mise en oeuvre a été éliminée. Ce retentat encore humide est utilisé directement pour l'étape de granulation.

### Granulation (étape (b) du procédé)

On ajoute, sur une durée d'environ 5 minutes, 48 g de cellulose microcristalline (Avicel PH 101) et 18 g de silice précipitée (Syloid 244 FP) tamisées au complexe fluoxétine/résine échangeuse de cations essoré introduit préalablement dans un mélangeur Kenwood « Chef » tournant à la vitesse minimale. Après environ 5 à 10 minutes, le mélange est séché en lit fluidisé (GPCG 1) jusqu'à un taux résiduel d'humidité d'environ 5 %. La poudre obtenue est hygroscopique et composée de particules relativement fines. Elle est soumise à un tamisage (500 µm) avant d'être enrobée.

### Enrobage (étape (c) du procédé)

On ajoute 165 g d'EUDRAGIT E100 dans 1287 g d'éthanol à 96 % et l'on agite jusqu'à dissolution complète du polymère, puis on ajoute sous agitation 18 g de silice précipité (Syloid 244 FP).

On transfère deux lots de granulés obtenus dans des conditions identiques de la manière décrite ci-dessus, dans la cuve d'enrobage d'un lit fluidisé GPCG 1 équipé d'un Würster et l'on pulvérise la solution d'enrobage avec une buse par le bas sur la masse fluidisée. La pression d'atomisation est réglée à une valeur relativement faible afin d'éviter le bouchage de la buse de pulvérisation par la poudré.

### Préparation des comprimés orodispersibles

On prépare par compression à sec des comprimés orodispersibles de 400 mg dosés à 20 mg de fluoxétine base. Ces comprimés ont la composition suivante :

| ingrédient | % en poids |
|---|---|
| Fluoxétine (chlorhydrate) | 5,59 |
| Résine échangeuse de cations faible (Amberlite IRP88) | 4, 61 |
| Cellulose microcristalline (Avicel PH 101) | 2, 68 |
| Silice précipitée | 2,51 |
| Eudragit E100 | 4,61 |
| **TOTAL granulés enrobés** | **26,57** |
| Mannitol poudre (Mannitol 60) | 17,75 |
| Mannitol granulé (Pearlitol SD200) | 41,18 |
| Crospovidone (Kollidon CL) | 8,00 |
| Aspartame | 4, 00 |
| Arôme Spearmint | 4,00 |
| Stéarate de magnésium | 1,50 |
| **TOTAL** | **100** |

La crospovidone, l'aspartame, la silice précipitée, l'arôme et le mannitol poudre sont pesés puis tamisés sur un tamis manuel de 1000 µm. Le mannitol granulé est également tamisé sur ce même tamis mais conservé dans un récipient à part. On mélange la crospovidone, l'aspartame, la silice précipitée, l'arôme et le mannitol en poudre et l'on ajoute au mélange les granulés de fluoxétine, puis le mannitol granulé. L'ensemble est mélangé dans un mélangeur cubique par retournement pendant 10 minutes à 14 tours/min. On ajoute ensuite le stéarate de magnésium, préalablement tamisé sur un tamis de 630 µm. On mélange de nouveau pendant 2 minutes à 14 tours/min.

Conditions de compression :
Poinçon : polo 11 mm
Masse : 400 mg
Dureté : 40 N
Epaisseur : environ 4 mm
Cadence : 10 000 - 15 000 cps/h
Régime fill-o-matic = 10 tours/min.

Le temps de désintégration des comprimés ainsi préparés, déterminé par un essai sur 6 comprimés, est compris entre 15 et 17 secondes. Ces comprimés satisfont aux spécifications de la monographie USP des comprimés de fluoxétine exigeant un taux de dissolution supérieur à 85 % en poids en 15 minutes dans une solution d'HCl 0,1 N.

Des tests en bouche réalisés ne révèlent ni amertume ni sensation de brûlure au niveau de la muqueuse buccale.

### Exemple 2

### Comprimés orodispersibles de rispéridone

On disperse, dans 200 g d'eau purifiée, 80 g de résine échangeuse de cations (Indion 204) et on agite avec un agitateur à hélice pendant 10 minutes jusqu'à obtention d'une suspension exempte de grumeaux. On ajoute 20 g de rispéridone et on agite le mélange pendant 1 heure à température ambiante. On granule le complexe rispéridone/résine obtenu avec un mélange de 278 g de lactose monohydrate et de 22 g de silice colloïdale (Aérosil 200) dans un mélangeur planétaire. On sèche la masse de granulation en lit fluidisé jusqu'à un taux d'humidité de 1 à 3 %. Après tamisage à travers un tamis de 40 mesh, on enrobe les granulés avec une solution aqueuse d'enrobage, préparée à partir de 41,0 g d'Eudragit EPO 2,88 g de laurylsulfate de sodium, 6,15 g de dibutylsebacatel et 255 g d'eau purifiée, en lit fluidisé par la technique de pulvérisation par le bas. Les granulés obtenus sont séchés dans le même lit fluidisé jusqu'à une teneur en humidité comprise entre 5 et 7 %, puis sont tamisés à travers un tamis de 35 mesh.

On prépare de manière analogue à celle décrite dans l'exemple 1 des comprimés orodispersibles de 400 mg dosés à 4 mg de rispéridone, ayant la composition suivante.

| ingrédient | % en poids |
|---|---|
| Granulés de rispéridone/résine enrobés | 22,5 |
| Mannitol poudre (Mannitol 60) | 21,5 |
| Mannitol granulé (Pearlitol SD200) | 40,5 |
| Polyplasdone XL | 10,0 |
| Aspartame | 2,00 |
| Aérosil 200 | 0,5 |
| Arôme menthe | 1,00 |
| Stéarate de magnésium | 2,50 |
| TOTAL | 100 |

Les comprimés ont un temps de désintégration dans la cavité buccale inférieur à 40 secondes. Lors de tests en bouche, ils ne présentent pas le moindre goût amer ni ne provoquent de sensation de brûlure dans la cavité buccale.

## Revendications

1. Granulés enrobés formés par un noyau comprenant un mélange
(A) d'au moins un principe actif pharmacologique aminé, de préférence sous forme de sel d'addition d'acide, ledit principe actif pharmacologique étant complexé par une résine échangeuse de cations faible comportant des groupes acide carboxylique (COO⁻), et
(B) d'au moins 15 % en poids, rapporté au poids total du complexe principe actif/résine échangeuse de cations faible, d'au moins un adsorbant hydrophile choisi dans le groupe consistant en la cellulose microcristalline, les amidons, le lactose, le lactose monohydrate, la silice colloïdale, la silice précipitée, et leurs mélanges,
ledit noyau comprenant le mélange des composants (A) et (B) étant enrobé par un polymère gastrosoluble.

2. Granulés selon la revendication 1, **caractérisés par le fait que** le principe actif pharmacologique aminé est un principe actif présentant un goût désagréable et/ou provoquant une sensation de brûlure lors de l'ingestion.

3. Granulés enrobés selon la revendication 1 ou 2, **caractérisés par le fait que** l'adsorbant hydrophile (B) comprend de la cellulose microcristalline et de la silice colloïdale.

4. Granulés enrobés selon l'une quelconque des revendications précédentes, **caractérisés par le fait que** l'adsorbant hydrophile (B) est présent à raison de 15 à 50 % en poids, de préférence à raison de 20 à 40 %, rapporté au poids total du complexe principe actif/résine échangeuse de cations.

5. Granulés enrobés selon la revendication 3 et 4, **caractérisés par le fait que** l'adsorbant hydrophile (B) comprend au moins 15 % en poids, rapporté au poids total du complexe principe actif/résine échangeuse de cations, de cellulose microcristalline et au moins 5 % en poids, rapporté au poids total du complexe principe actif/résine échangeuse de cations, de silice colloïdale.

6. Granulés enrobés selon l'une quelconque des revendications précédentes, **caractérisés par le fait que** le principe actif pharmacologique est choisi dans le groupe constitué de fluoxétine et de rispéridone, de préférence de sels d'addition d'acide de ces composés.

7. Granulés enrobés selon la revendication 6, **caractérisés par le fait que** le sel d'addition d'acide est un chlorhydrate.

8. Granulés enrobés selon l'une quelconque des revendications précédentes, **caractérisés par le fait que** la résine échangeuse de cations faibles est un poly(acide méthacrylique) réticulé, de préférence un poly(acide méthacrylique) réticulé par du divinylbenzène.

9. Comprimé orodispersible comprenant des granulés enrobés selon l'une quelconque des revendications précédentes et un ou plusieurs adjuvants choisis parmi les agents de désintégration, les diluants, les excipients pour compression directe, les agents d'écoulement, les lubrifiants, les édulcorants et les arômes.

10. Procédé de fabrication de granulés enrobés selon l'une quelconque des revendications 1 à 8, comprenant,
(a) la complexation, en milieu aqueux, d'au moins un principe actif pharmacologique aminé par une résine échangeuse de cations faible comportant des groupes carboxyle (-COO⁻),
(b) la granulation du complexe principe actif/résine obtenu contenant encore au moins une partie de l'eau utilisée pour la complexation, en présence d'au moins 15 % en poids, de préférence de 15 à 50 % en poids, rapporté au poids total du complexe principe actif/résine, d'au moins un adsorbant hydrophile choisi dans le groupe consistant en la cellulose microcristalline, les amidons, le lactose, le lactose monohydrate, la silice colloïdale, la silice précipitée et leurs mélanges, et
(c) l'enrobage des granulés obtenus à l'étape (b) par au moins un polymère gastrosoluble.

11. Procédé selon la revendication 10, **caractérisé en ce qu'**il comporte en outre, à l'issue de l'étape (a) de complexation, une étape d'essorage du complexe principe actif/résine ainsi obtenu.

## Claims

1. Coated granules formed by a core comprising a mixture:
(A) of at least one aminated pharmacological active principle, preferably in the form of an acid addition salt, said pharmacological active principle being complexed by a weak cation-exchange resin comprising carboxylic acid groups (COO⁻); and
(B) of at least 15% by weight, relative to the total weight of the active principle/weak cation-exchange resin complex, of at least one hydrophilic adsorbent chosen in the group consisting of microcrystalline cellulose, starches, lactose, lactose monohydrate, colloidal silica, precipitated silica and their mixtures,
said core comprising the mixture of components (A) and (B) being coated by a gastrosoluble polymer.

2. The granules as claimed in claim 1, **characterized by** the fact that the aminated pharmacological active principle is an active principle having an unpleasant taste and/or causing a burning sensation during ingestion.

3. The coated granules as claimed in claim 1 or 2, **characterized by** the fact that the hydrophilic adsorbent (B) comprises microcrystalline cellulose and colloidal silica.

4. The coated granules as claimed in any one of the preceding claims, **characterized by** the fact that the hydrophilic adsorbent (B) is present in an amount of 15 to 50% by weight, preferably in an amount of 20 to 40%, relative to the total weight of the active principle/cation-exchange resin complex.

5. The coated granules as claimed in claim 3 and 4, **characterized by** the fact that the hydrophilic adsorbent (B) comprises at least 15% by weight, relative to the total weight of the active principle/cation-exchange resin complex, of microcrystalline cellulose and at least 5% by weight, relative to the total weight of the active principle/cation-exchange resin complex, of colloidal silica.

6. The coated granules as claimed in any one of the preceding claims, **characterized by** the fact that the pharmacological active principle is chosen from the group consisting of fluoxetine and risperidone, preferably of acid addition salts of these compounds.

7. The coated granules as claimed in claim 6, **characterized by** the fact that the acid addition salt is a hydrochloride.

8. The coated granules as claimed in any one of the preceding claims, **characterized by** the fact that the weak cation-exchange resin is a crosslinked polymethacrylic acid, preferably a polymethacrylic acid crosslinked by divinylbenzene.

9. An orodispersible tablet comprising coated granules as claimed in any one of the preceding claims and one or more adjuvants chosen from disintegrants, diluents, excipients for direct compression, flow agents, lubricants, sweeteners and flavors.

10. A process for manufacturing coated granules as claimed in any one of claims 1 to 8, comprising:
(a) the complexation, in an aqueous medium, of at least one aminated pharmacological active principle by a weak cation-exchange resin comprising carboxyl groups (-COO⁻);
(b) the granulation of the active principle/resin complex obtained still containing at least some of the water used for the complexation, in the presence of at least 15% by weight, preferably 15 to 50% by weight, relative to the total weight of the active principle/resin complex, of at least one hydrophilic adsorbent chosen in the group consisting of microcrystalline cellulose, starches, lactose, lactose monohydrate, colloidal silica, precipitated silica and their mixtures, and
(c) the coating of the granules obtained in step (b) by at least one gastrosoluble polymer.

11. The process according to claim 10, **characterized in that** it comprises moreover, at the end of the complexation step (a), a hydroextraction step of the active principle/resin complex thus obtained.

## Patentansprüche

1. Überzogene Granalien, gebildet aus einem Kern, umfassend ein Gemisch aus
(A) mindestens einem pharmakologisch wirksamen Amin-Wirkstoff, vorzugsweise in Form eines Säurezugabesalzes, wobei der pharmakologische Wirkstoff durch ein schwaches Kationenaustauscherharz, das Carbonsäuregruppen (COO⁻) aufweist, komplexiert ist, und
(B) mindestens 15 Gew.-%, bezogen auf das Gesamtgewicht des Komplexes aus Wirkstoff/schwaches Kationenaustauscherharz, mindestens eines hydrophilen Adsorptionsmittels, ausgewählt aus der Gruppe, bestehend aus mikrokristalliner Cellulose, den Stärken, der Lactose, dem Lactosemonohydrat, dem kolloidalen Siliciumdioxid, präzipitiertem Siliciumdioxid, und ihren Gemischen,
wobei der Kern das Gemisch der Komponenten (A) und (B) umfasst, das durch ein magenlösliches Polymer überzogen ist.

2. Granalien nach Anspruch 1, **dadurch gekennzeichnet, dass** der pharmakologische Amin-Wirkstoff ein Wirkstoff ist der einen unangenehmen Geschmack aufweist und/oder ein brennendes Gefühl während der Einnahme hervorruft.

3. Überzogene Granalien nach Anspruch 1 oder 2, **dadurch gekennzeichnet, dass** das hydrophile Adsorptionsmittel (B) mikrokristalline Cellulose und kolloidales Siliciumdioxid umfasst.

4. Überzogene Granalien nach einem der vorhergehenden Ansprüche, **dadurch gekennzeichnet, dass** das hydrophile Adsorptionsmittel (B) in 15 bis 50 Gew.-%, vorzugsweise in 20 bis 40 Gew.-%, bezogen auf das Gesamtgewicht des Komplexes aus Wirkstoff/Kationenaustauscherharz vorliegt.

5. Überzogene Granalien nach Anspruch 3 und 4, **dadurch gekennzeichnet, dass** das hydrophile Adsorptionsmittel (B) mindestens 15 Gew.-%, bezogen auf das Gesamtgewicht des Komplexes aus Wirkstoff/Kationenaustauscherharz, mikrokristalline Cellulose und mindestens 5 Gew.-%, bezogen auf das Gesamtgewicht des Komplexes aus Wirkstoff/ Kationenaustauscherharz, kolloidales Siliciumdioxid umfasst.

6. Überzogene Granalien nach einem der vorhergehenden Ansprüche, **dadurch gekennzeichnet, dass** der pharmakologische Wirkstoff ausgewählt ist aus der Gruppe, bestehend aus Fluoxetin und Risperidon, vorzugsweise aus Säurezugabesalzen dieser Komponenten.

7. Überzogene Granalien nach Anspruch 6, **dadurch gekennzeichnet, dass** das Säurezugabesalz ein Hydrochlorid ist.

8. Überzogene Granalien nach einem der vorhergehenden Ansprüche, **dadurch gekennzeichnet, dass** das schwache Kationenaustauscherharz eine vernetzte Poly(methacrylsäure), vorzugsweise eine durch Divinylbenzol vernetzte Poly(methacrylsäure) ist.

9. Schmelztablette, umfassend überzogene Granalien nach einem der vorhergehenden Ansprüche und ein oder mehrere Adjuvantien, ausgewählt aus den Desintegrationsmitteln, den Verdünnungsmitteln, den Exzipienten für direkte Pressung, den Fließmitteln, den Gleitmitteln, Süßmitteln und den Aromen.

10. Verfahren zur Herstellung von überzogenen Granalien nach einem der Ansprüche 1 bis 8, umfassend:
(a) die Komplexierung mindestens eines pharmakologischen Amin-Wirkstoffs in wässrigem Milieu durch ein schwaches Kationenaustauscherharz, das Carboxylgruppen (COO⁻) trägt,
(b) Granulierung des erhaltenen Komplexes aus Wirkstoff/Harz, außerdem enthaltend mindestens einen Teil des zur Komplexierung verwendeten Wassers, in Gegenwart von mindestens 15 Gew.-%, vorzugsweise 15 bis 50 Gew.-%, bezogen auf das Gesamtgewicht des Komplexes aus Wirkstoff/Harz, mindestens eines hydrophilen Adsorptionsmittels, ausgewählt aus der Gruppe bestehend aus mikrokristalliner Cellulose, den Stärken, der Lactose, dem Lactosemonohydrat, dem kolloidalen Siliciumdioxid, präzipitiertem Siliciumdioxid, und ihren Gemischen, und
(c) die Überziehen der in Schritt (b) erhaltenen Granalien durch mindestens ein magenlösliches Polymer.

11. Verfahren nach Anspruch 10, **dadurch gekennzeichnet, dass** es weiterhin nach Schritt (a) der Komplexierung einen Schleuderschritt des so erhaltenen Komplexes aus Wirkstoff/Harz umfasst.
